Europäisches Patentamt

European Patent Office

Office européen des brevets

(19)

(11) Publication number: **0 173 457**
**A1**

## (12) EUROPEAN PATENT APPLICATION

(21) Application number: **85305330.4**

(22) Date of filing: **25.07.85**

(51) Int. Cl.⁴: **C 07 C 125/06**

(30) Priority: **02.08.84 GB 8419763**

(43) Date of publication of application: **05.03.86**
**Bulletin 86/10**

(84) Designated Contracting States: **BE DE FR GB IT NL**

(71) Applicant: **The British Petroleum Company p.l.c.,**
**Britannic House Moor Lane, London EC2Y 9BU (GB)**

(72) Inventor: **Alper, Howard, 17 Winlock Crescent, Ottawa**
**K2G 3X5 (CA)**
Inventor: **Smith, David John Harry, British Petroleum**
**Company p.l.c. Chertsey Road, Sunbury-on-Thames**
**Middlesex, TW16 7LN (GB)**

(74) Representative: **Fawcett, Richard Fennelly et al, BP**
**INTERNATIONAL LIMITED Patents Division Chertsey**
**Road, Sunbury-on-Thames Middlesex, TW16 7LN (GB)**

(54) **Process for the production of carbamate esters.**

(57) Carbamate esters are prepared by contacting an aromatic amine, carbon monoxide, an alcohol and a protonic acid in the presence of a catalyst comprising (a) a metal selected from palladium, rhodium, ruthenium, iridium and cobalt and (b) copper.

1

## PROCESS FOR THE PRODUCTION OF CARBAMATE ESTERS

The present invention relates in general to a process for the production of carbamate esters and in particular to a process for the production of carbamate esters by the catalysed reaction of an aromatic amine, carbon monoxide and an alcohol, optionally in the presence of oxygen.

Processes for the production of carboxylic acid esters by reacting an olefin with carbon monoxide and an alcohol in the presence of a catalyst and in the presence or absence of oxygen are known. Representative of the published art are US Patent No. 4303589, Belgian Patent No. 877770, Japanese Patent Publication No. 53040709 and US Patent No. 3780074.

US Patent No. 4303589 (Monsanto) describes a process for the production of carboxylate esters by (a) reacting internal olefins with carbon monoxide and an alcohol at 170 to 200°C and 1200-1800 psig in the presence of a cobalt catalyst and a pyridine promoter, (b) diluting the reaction mixture with a large amount of hydrocarbon to cause phase separation, (c) separating the ester from the other phase, which contains more than 90% of the cobalt catalyst and (d) recycling the catalyst to step (a).

Belgian Patent No. 877770 describes the production of polycarboxylic esters by reacting an olefin containing at least two conjugated double bonds with carbon monoxide and an alcohol in the presence of a base and a palladium/copper catalyst.

Japanese Patent Publication No. 53040709 describes the production of dicarboxylic acid diesters by reacting an olefin, carbon monoxide, oxygen and an alcohol in the presence of a catalyst containing (a) a palladium group metal or a compound thereof, (b) a copper salt and (c) a tertiary amine.

Finally, USP 3,780,075 describes the production of alkadienoic acid esters by reacting a 4-12 carbon acyclic conjugated aliphatic diolefin with a 1 to 20 carbon monohydroxy alcohol and carbon monoxide in the presence of zerovalent palladium and a phosphine activator at 80 to 160°C in the absence of oxygen.

Methods are also known for the hydroesterification of acetylene to produce isomeric esters. For example, G.P. Chiusli et al report in Chem. Ind., 977, (1968) the reaction of acetylene with carbon monoxide in the presence of 4% oxygen and thiourea and a palladium (II) chloride catalyst. A disadvantage of this process is that the selectivity to isomeric esters (cis and trans-esters) is considerably reduced by the accompanying formation of polymeric materials and isomeric muconate esters.

Our copending European application publication No. 105704 discloses a process for the production of a carboxylic acid ester which process comprises reacting an unsaturated hydrocarbon with carbon monoxide and an alcohol in the presence of a protonic acid and as a catalyst (a) at least one of the metals palladium, rhodium, ruthenium, iridium and cobalt, and (b) copper.

We have now found that by using an aromatic amine instead of an unsaturated hydrocarbon in the process of EP-A-105704, carbamate esters can be produced from alcohols.

Aromatic carbamate esters are convertible by reaction with formaldehyde into urethanes and/or polyurethanes and are thermally decomposable to isocyanates which are intermediates in the formation of urethanes and polyurethanes by reaction with monohydric alcohols and polyols respectively. The process therefore offers an alternative route to the production of aromatic isocyanates and ultimately polyurethanes which avoids the use of phosgene as conventionally employed for converting aromatic amines, for example diaminotoluenes, to isocyanates.

Accordingly, the present invention provides a process for the

0173457

production of carbamate esters which process comprises reacting an aromatic amine with carbon monoxide and an alcohol in the presence of a protonic acid and a catalyst comprising (a) at least one of the metals selected from the group palladium, rhodium, ruthenium, iridium and cobalt, and (b) copper.

The process of the present invention can be operated under remarkably mild conditions, for example room temperature and one atmosphere pressure.

The aromatic amine constituting one of the reactants in the process of the invention may suitably be aniline or a derivative thereof, an aminotoluene, a diarylamine, a phenylene diamine (benzene diamine) or any other suitable aromatic amine. Suitable derivatives of aniline include the C-alkyl substituted, for example toluidines and xylidenes; the C-alkoxy substituted, for example anisidines, phenetidines and cresidines; and the haloanilines, for examples chloroaniline and nitroanilines. Preferred aminotoluenes are the diaminotoluenes, of which the 2,4- and 2,6- isomers, or mixtures thereof, are particularly preferred. A suitable phenylene diamine is 4,4'-methylene dianiline. The preferred aromatic amines are aniline, aminotoluene, diarylamine and phenylene diamine.

The carbon monoxide may be provided by any suitable source. The carbon monoxide pressure may suitably be the autogenous pressure at the reaction temperature employed. Alternatively, elevated pressures, suitably in the range from 2 to 250 psig above the autogenous pressure at the reaction temperature may be employed.

As regards the alcohol reactant, monohydric and polyhydric alcohols may be employed. Suitable alcohols may be represented by the formula $R_2CHOH$ wherein R is independently hydrogen, alkyl, aryl or hydroxyalkyl, or the two R groups together form a ring. Preferably, the alcohol is an alkanol. Examples of suitable alcohols include methanol, ethanol, propanols, butanols, pentanols, hexanols, for example 2-ethylhexanol, benzyl alcohol and 1,4-butanediol. The amount of alcohol employed may suitably be at least the stoichiometric amount required to react with the aromatic amine. It is preferred, however, to employ a substantial excess of alcohol over the stoichiometric amount, the alcohol then performing the dual role of reactant and diluent for the reaction.

The protonic acid may be either a mineral acid, preferably hydrochloric acid or sulphuric acid, or an organic acid such as acetic acid, carbonate acid or other carboxylic acids.

With regard to the catalyst, one or more of the metals palladium, rhodium, ruthenium, iridium and cobalt is employed as component (a). The metal(s) may be in the form of the elemental metal(s), such as a finely divided powder, or in the form of a compound of the metal(s). Suitable compounds of the metal(s) include the chloride, iodides, acetates and nitrates, preferably the chlorides. Preferably the metal is palladium, suitably in the form of palladium (II) chloride.

Copper, which constitutes component (b) of the catalyst may suitably be added as a cuprous or a cupric compound or as a mixture thereof. A wide variety of copper compounds may be used in the process of the invention. Examples of suitable copper compounds include but are not limited to copper (I) acetate, copper (II) acetylacetonate, copper (I) bromide, copper (I) chloride, copper (II) chloride, copper (I) iodide, copper (II) nitrate, and the like.

As regards the ratios of the catalyst components, the molar ratio of copper component (b) to metal(s) component (a) may suitably be in the range from 1:1 to 200:1, preferably from 2:1 to 50:1.

The molar ratio of aromatic amine to the metal(s) component (a) may suitably be in the range from 5:1 to 1000:1, preferably from 10:1 to 250:1.

Oxygen may be present or absent. However, it is preferred to operate in the presence of oxygen because by doing so the product yields can be improved. Oxygen may be supplied to the reaction either as essentially pure oxygen or admixed with other gases which are substantially inert under the reaction conditions. Air may conveniently be used as the source of oxygen. The oxygen pressure may suitably be the autogenous pressure at the reaction temperature employed. Alternatively elevated pressures may be employed if desired.

A supplemental solvent may be employed if desired. The particular solvent employed may form a single phase with the alcohol reactant. Alternatively a solvent which is capable of forming a second liquid phase may be employed. The particular solvent employed should be inert under the reaction conditions. Suitable solvents which form a single phase with the alcohol reactant include oxygenated hydrocarbons, for example tetrahydrofuran. Suitable solvents capable of forming a second liquid phase include aliphatic hydrocarbons, cycloaliphatic hydrocarbons, aromatic hydrocarbons, alkyl-substituted aromatic hydrocarbons or halogenated aliphatic or aromatic hydrocarbons. Examples of suitable solvents capable of forming a second liquid phase include benzene, toluene, hexane, cyclohexane, chlorobenzene, bromobenzene, a xylene, dichloromethane, chloroform and 1,2-dichloroethane. It will be appreciated by those skilled in the art that the organic solvent should be chosen having regard to the difference in boiling points between the products of the reaction and the solvent so as to facilitate separation of the reaction mixture into its individual components. The amount of supplemental solvent based on the olefin reactant may vary over a wide range, suitably from 20 to 0.2, preferably from 5 to 1, volumes of supplemental solvent per volume of olefin reactant.

Using solvents which are capable of forming a second liquid phase it may be advantageous to employ a surfactant. Typical of the surfactants which may be used are quaternary ammonium salts, for example cetyltrimethylammonium bromide, alkali metal sulphates, alkali metal salts of alkaryl sulphonic acids, alkali metal salts of alkanoic acids and 1-alkyl pyridinium salts.

The process may suitably and preferably be operated at ambient temperature, although elevated temperatures, for example in the range 20 to 150°C or even higher may be employed. The reaction time may vary over a wide range, suitably from about 30 minutes to 8 hours, though longer reaction times may be employed if desired.

The process may be carried out batchwise or continously, preferably continously.

The invention will now be described in greater detail by reference to the following Examples. However, these examples are provided only to illustrate the present invention which includes equivalent embodiments, variations and modifications.

PROCESS OPERATED IN THE PRESENCE OF OXYGEN

Example 1

Palladium (II) chloride (0.1g; 0.56 mmol) was added to methanol (50 ml) through which was bubbled carbon monoxide (1 atmosphere). After 1 minute conc. hydrochloric acid (0.5 ml) was added. When the solution turned yellow (indicating that the palladium chloride had dissolved), copper (II) chloride (0.5g; 3.7 mmol) was added and oxygen (1 atmosphere) was bubbled through the solution in addition to the carbon monoxide. p-Toluidine (6 mmol) was then added and thereaction mixture was stirred for 4 hours at 25°C. After 2 hours a further 0.5 ml conc. hydrochloric acid was added.

The reaction product was then extracted with hexane and the hexane evaporated to give a pure product which was identified as the carbamate ester of formula $p-CH_3C_6H_4NHCOOCH_3$. The results are shown in Table 1.

Examples 2 - 12

The procedure of Example 1 was repeated except that the reactants or catalyst were changed as shown in Table 1. The results are reported in Table 1.

Table 1

Products Obtained from the Reaction of aromatic amine with $CO/O_2/$ alcohol/HCL/$PdCl_2$/$CuCl_2$

| Example | Amine $RNH_2$ where R = | Alcohol | Product | Yield, % |
|---|---|---|---|---|
| 1 | $p-CH_3C_6H_4$ | $CH_3OH$ | $p-CH_3C_6H_4NHCOOCH_3$ | 68 |
| 2 | " | $CH_3OH$ (no $O_2$) | $P-CH_3C_6H_4NHCOOCH_3$ | 34[a] |
| 3 | " | $CH_3OH[Pd(OAc)_2/Cu(OAc)_2]$ | $p-CH_3C_6H_4NHCOOCH_3$ | 91 |
| 4 | " | $C_2H_5OH$ | $p-CH_3C_6H_4NHCOOC_2H_5$ | 51 |
| 5 | Ph[b] | $CH_3OH$ | $PhNHCOOCH_3$ | 99 |
| 6 | Ph[b] | $C_2H_5OH$ | $PhNHCOOC_2H_5$ | 64 |
| 7 | $p-ClC_6H_4$ | $CH_3OH$ | $p-ClC_6H_4NHCOOCH_3$ | 61[a] |
| 8 | $m-CH_3COC_6H_4$ | $CH_3OH$ | $m-CH_3COC_6H_4NHCOOCH_3$ | 99 |
| 9 | $2,5-(CH_3)_2C_6H_3$ | $CH_3OH$ | $2,5-(CH_3)_2C_6H_3NHCOOCH_3$ | 70 |
| 10 | $3,5-(CH_3)_2C_6H_3$ | $CH_3OH$ | $3,5-(CH_3)_2C_6H_3NHCOOCH_3$ | 23 |
| 11 | $2,6-(CH_3)_2C_6H_3$ | $CH_3OH$ | $2,4-(CH_3)_2C_6H_3 NHCOOCH_3$ | 16 |

[a] An unidentified by-product was also formed.

[b] = phenyl

Claims:

1.  A process for the production of carbamate esters comprising reacting an aromatic amine with carbon monoxide and an alcohol in the presence of a protonic acid and a catalyst comprising (a) at least one of the metal selected from the group palladium, rhodium, ruthenium, iridium and cobalt and (b) copper.

2.  The process of Claim 1 wherein the protonic acid is a mineral acid.

3.  The process of any of the proceeding claims wherein the mineral acid is selected from hydrochloric acid and sulphuric acid.

4.  The process of any of the proceedings claims wherein the molar ratio of copper to metal in the range from 1:1 to 200:1.

5.  The process of any of the proceeding claims wherein the molar ratio of aromatic amine to the metal is in the range from 5:1 to 1000:1.

6.  The process of any of the proceeding claims wherein oxygen is present.

7.  The process of any of the proceeding claims wherein the process is operated at room temperature and 1 atomsphere pressure.

**European Patent Office**

**EUROPEAN SEARCH REPORT**

Application number

EP 85 30 5330

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | DE-A-2 908 251 (BAYER) * claim 1, page 19, second paragraph * | 1,6 | C 07 C 125/06 |
| A | DE-B-2 603 574 (MITSUI TOATSU) | 1 | |
| A | US-A-4 266 070 (HALCON RESEARCH) | 1 | |
| A | US-A-4 260 781 (HALCON RESEARCH) | 1 | |
| A | US-A-4 251 667 (ATLANTIC RICHFIELD) | 1 | |
| A | WO-A-8 002 686 (MITSUI TOATSU) | 1 | TECHNICAL FIELDS SEARCHED (Int. Cl.4) |
| A | PATENT ABSTRACTS OF JAPAN, vol. 7, no. 46, (C-153)[1191], 23 th February 1983; & JP - A - 57 200 349 (ASAHI KASEI KOGYO) 08-12-1982 | 1 | C 07 C 125/06 C 07 C 125/065 C 07 C 125/063 |
| D,A | EP-A-0 105 704 (BP) | 1 | |

The present search report has been drawn up for all claims

| Place of search BERLIN | Date of completion of the search 29-10-1985 | Examiner KAPTEYN H G |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82